# EUROPEAN PATENT SPECIFICATION

(11) **EP 0 677 281 B1**
(45) Date of publication and mention of the grant of the patent: **16.02.2000**
(21) Application number: 95830097.2
(22) Date of filing: 17.03.1995
(51) Int. Cl.: A61F 2/36

(54) **Femoral prosthesis for recovering a prosthesis implantation which has provided negative results**
Femurprothese zum Wiederherstellen einer Prothesenimplantation, die zu negativen Ergebnissen geführt hat
Prothèse fémorale pour la restauration d'une implantation prothétique ayant conduit à des résultats négatifs

(30) Priority: 17.03.1994 IT MI940496
(43) Date of publication of application: 18.10.1995
(73) Proprietor: CREMASCOLI ORTHO S.r.l., Mantova (IT)
(72) Inventor: Cremascoli, Patrizio, c/o G. Cremascoli S.r.l., Mantova (IT)
(74) Representative: Cicogna, Franco

(56) References cited:
- EP-A- 0 159 462
- EP-A- 0 310 566
- EP-A- 0 462 357
- EP-A- 0 567 349
- EP-A- 0 634 154
- WO-A-91/18563
- DE-A- 3 829 361
- DE-A- 4 320 086
- FR-A- 2 629 707
- FR-A- 2 640 497

## Description

The present invention relates to a femoral prosthesis, which has been particularly designed for recovering prosthesis implantations which have provided negative results.

As is known, prior femoral prostheses are conventionally made in a single body.

This construction does not allow an operation staff to properly fit the prosthesis to a patient, mainly in those cases in which it is necessary to modify or recover prosthesis implantations which have provided a negative result for not having had success.

### SUMMARY OF THE INVENTION

Accordingly, the aim of the present invention is to solve the above mentioned problem, by providing a femoral prosthesis, made of modular elements, which can be perfectly fitted to the different femoral patterns of the patients, mainly in those cases in which it is necessary to replace existing prostheses, which have provided a negative result.

Within the scope of the above mentioned aim, a main object of the present invention is to provide such a femoral prosthesis, which can be perfectly anchored to the femoral bone, and is not susceptible to undesired rotary movements and is moreover adapted to improve the primary anchoring thereof to the femoral bone.

Another object of the present invention is to provide such a femoral prosthesis which is very reliable and safe in operation.

Yet another object of the present invention is to provide such a femoral prosthesis which can be quickly and firmly coupled to the implements which are used for introducing the prosthesis into the femoral bone and removing it from said bone.

According to one aspect of the present invention, the above mentioned aim and objects, as well as yet other objects, which will become more apparent hereinafter, are achieved by a femoral prosthesis as defined in claim 1.

### BRIEF DESCRIPTION OF THE DRAWINGS

Further characteristics and advantages of the present invention will become more apparent hereinafter from the following detailed disclosure of a preferred, though not exclusive, embodiment of a femoral prosthesis, which has been specifically designed for recovering prosthesis implantations which have provided negative results, and which is illustrated, by way of an indicative, but not limitative example, in the accompanying drawings, where:

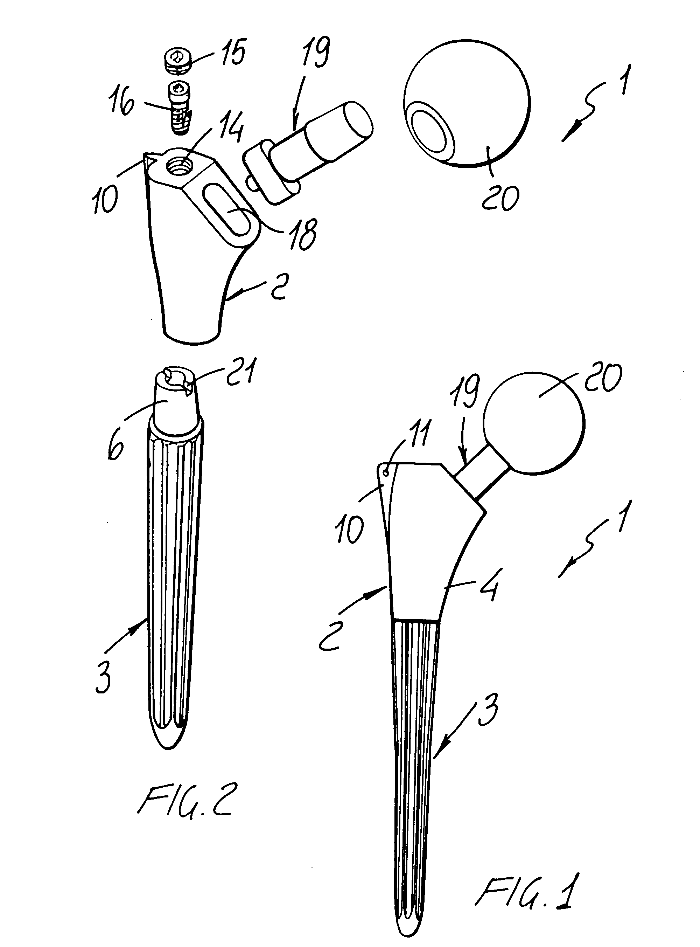

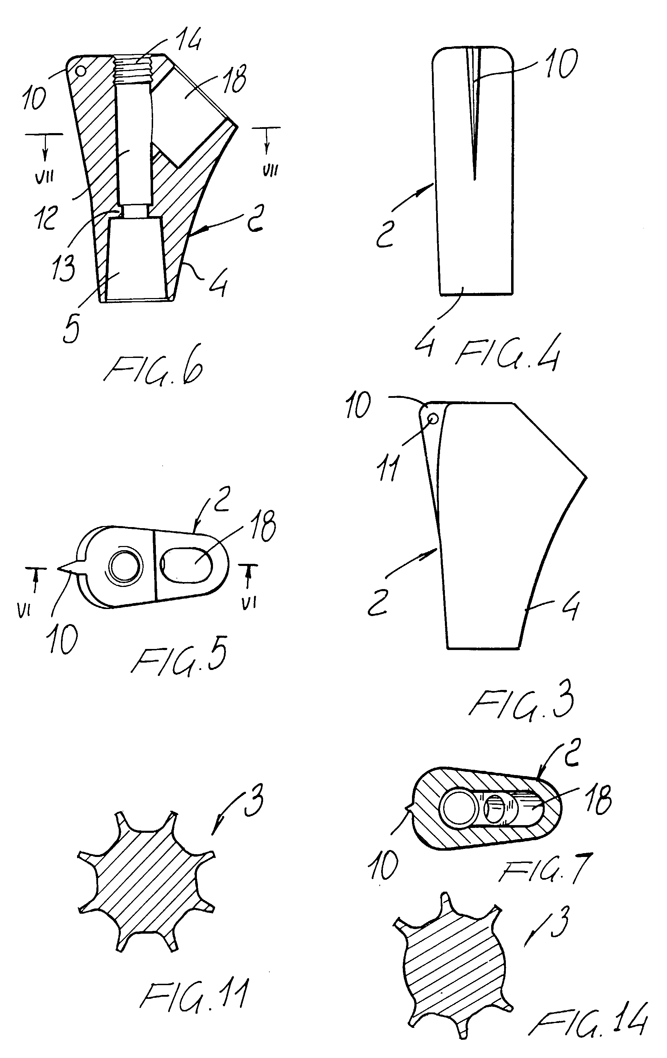

Figure 1 is a front elevation view illustrating the subject femoral prosthesis in an assembled condition thereof;
Figure 2 is an exploded perspective view illustrating the femoral prosthesis according to the invention;
Figure 3 is a front elevation view illustrating the proximal body of the prosthesis;
Figure 4 is a further side elevation view illustrating the proximal body of the prosthesis;
Figure 5 is a top plan view illustrating the proximal body of the prosthesis;
Figure 6 is a cross-sectional view, substantially taken along the section line VI-VI of Figure 5;
Figure 7 is a further cross-sectional view, substantially taken along the section line VII-VII of Figure 6;
Figures 8, 9 and 10 illustrate the distal body or stem of the prosthesis made with three different lengths;

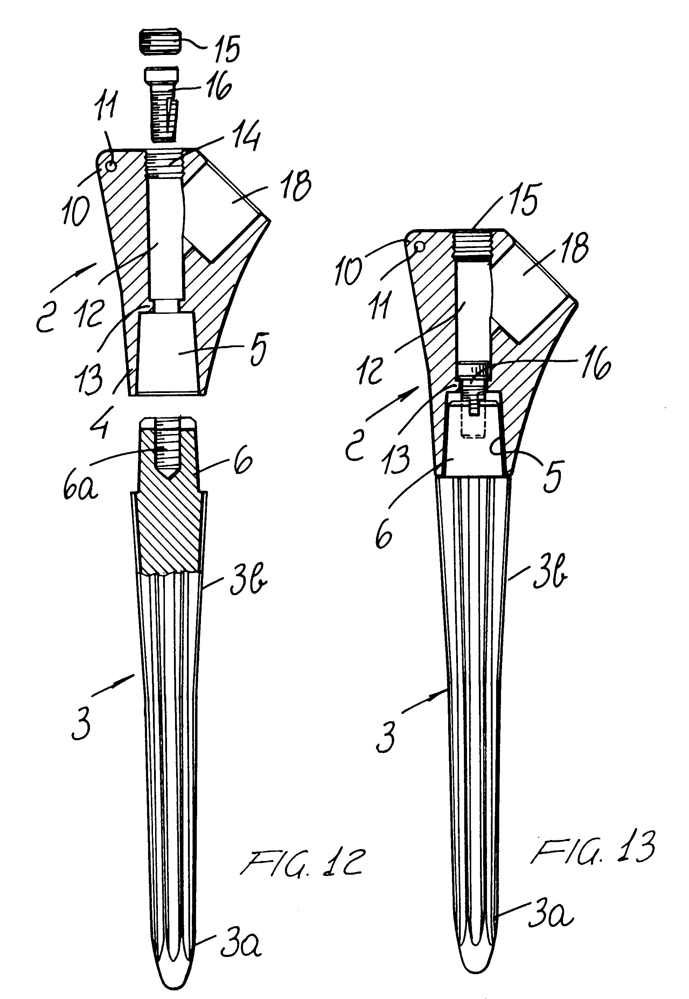

Figure 11 is a cross-sectional view substantially taken along the line XI-XI of Figure 10;
Figure 12 is a cross-sectional exploded view of the femoral prosthesis;
Figure 13 illustrates the subject prosthesis in an assembled condition, the proximal body being shown in cross-section; and
Figure 14 is a further cross-sectional view substantially taken along the line XII-XII of Figure 8.

### DESCRIPTION OF THE PREFERRED EMBODIMENTS

With reference to the number references of the above mentioned figures, the subject femoral prosthesis, which has been specifically designed for recovering prosthesis implantations or systems which have provided negative results, which has been generally indicated by the reference number 1, comprises a proximal body 2 and a stem 3, which can be removably coupled to the proximal body.

The proximal body 2 is provided, at the proximal end portion thereof, with a substantially elliptical cross-section configuration, which is changed or reduced toward the distal tip portion, indicated at 4, where it has a substantial circular configuration.

At the tip portion 4, there is provided a conic seat or recess 5, in which the conic end-piece 6 of the distal body or stem 3 can be engaged.

The body 2 is provided, at the proximal portion thereof, and in its side region, with a substantially triangular cross-section wing 10, the function of which is that of preventing the prosthesis from rotating and improving the primary anchoring thereof.

Moreover, at the wing 10, there is provided a throughgoing hole 11, allowing to affix the muscle fascia.

Inside the body 2, and aligned with the conic seat 5, is provided a throughgoing channel 12 which, toward the distal end portion thereof, is delimited by an abutment 13, whereas on the top a threaded seat 14 is provided.

In the threaded seat 14 a plug 15 can be engaged, said plug preventing possible physiologic liquids from entering, the connection with the stem 3 being performed by a screw 16, engaging in the channel 12 and having the head thereof abutting against the abutment 13.

At the medial proximal region there is provided an oval seat 18, in which can be engaged the neck 19 to which a head 20 can be connected.

The proximal body 2 is provided in several sizes.

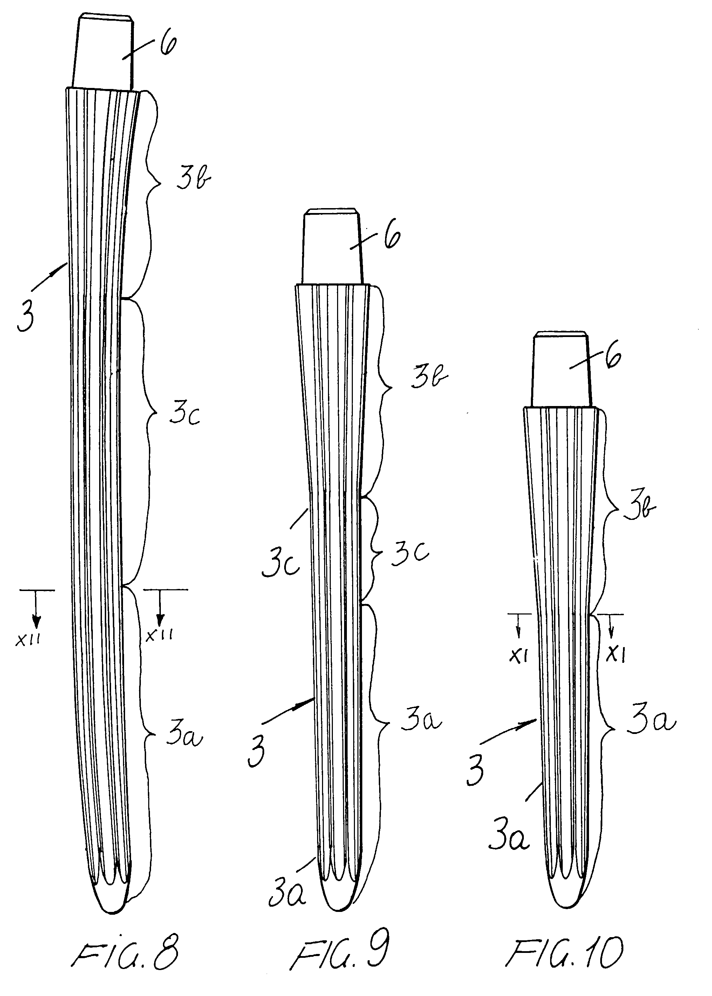

The distal body or stem, in turn, is made with different lengths, as is clearly shown in Figures 8 to 10.

Each length, moreover, is available with different sizes.

A main feature of the invention is that the stem is provided, in its cross-section, with a substantially star-like configuration.

On the proximal tip portion there is provided the conic end- piece 6, which is provided with a threaded hole 6a in which the screw 16 is engaged.

On the top surface on the conic end-piece, there is moreover provided a cross slot 21 which allows,by using a suitable implement, to properly locate the stem inside the femoral canal.

The stems are made by providing a conic distal portion, indicated at 3a and possibly a proximal portion 3b, which, the size being the same, is the same for all of the lengths.

If necessary, is moreover provided a cylindrical portion 3c having different lengths.

Moreover, the larger length stems are so bent as to allow them to be perfectly fitted to the curvature of the femoral diaphysis, as is schematically shown in Figure 8.

In order to facilitate the introduction of the larger length stems inside the femoral canal, it is provided that along the longitudinal extension of the stems, the substantially star-like cross section is lacking of the star points arranged on the convex and concave portions of the stems, as clearly shown in Figure 14.

Owing to the disclosed construction, a femoral prosthesis has been provided which can be made by modular elements which can be easily assembled so as to allow the operating staff to choose the optimal femoral prosthesis size, depending on the pattern of the patient femoral bone.

Moreover, the provision of easily and quickly assembleable modular elements allow to greatly facilitate the implantation of the prosthesis.

The invention as disclosed is susceptible to several modifications and variations, all of which will come within the scope of the inventive idea.

Moreover, all the details can be replaced by other technically equivalent elements.

In practicing the invention, the used materials, as well as the contingent size and shapes, can be any, depending on requirements.

## Claims

1. A femoral prosthesis (1), which has been specifically designed for recovering prosthesis implantations which have provided negative results, said femoral prosthesis comprising a proximal body (2), and a distal body or stem (3) having a substantially star-like cross-section and the size of the proximal portion (3b) of the stem being the same for all of the lengths of the stem, said proximal body (2) having, at a proximal end portion thereof, a substantially elliptical cross-section which is changed to a substantially circular configuration toward the distal tip portion (4), characterized in that in said distal tip portion (4) a conic seat (5) is provided for removably coupling said proximal body (2) with a conic end-piece (6) of the stem, and that, depending on the different lengths of the stem, there are provided cylindrical central portions (3c) having different lengths.

2. A femoral prosthesis, according to Claim 1, characterized in that the larger length stems (3) are so bent as to be precisely fitted to the curvature of the femoral diaphysis.

3. A femoral prosthesis, according to Claims 1 and 2, characterized in that along the overall extension of said larger length stems (3), said substantially star-like cross-section is lacking of one or more points arranged on the convex and concave portions of said stems.

4. A femoral prosthesis, according to Claims 1 to 3, characterized in that said proximal body is provided, at the proximal-side region thereof, with a wing (10) having a substantially triangular cross-section, and extending for a longitudinal portion.

5. A femoral prosthesis, according to Claims 1 to 4, characterized in that said wing (10) is provided with a throughgoing hole (11).

6. A femoral prosthesis, according to one or more of the preceding claims, characterized in that said prosthesis further comprises, through the proximal body (2), on the extension of said conic seat (5), a throughgoing channel (12) for engaging therein a screw (16) for the connection with the end-piece (6) of the stem (3).

7. A femoral prosthesis, according to one or more of the preceding claims, characterized in that said channel (12), at the proximal end portion thereof, is provided with a threaded seat (14) for engaging therein a plug (15).

8. A femoral prosthesis, according to one or more of the preceding claims, characterized in that said proximal body (2) is provided, in the medial-proximal region thereof, with an oval seat (18) for the connection with a neck (19) in turn connected with a head (20).

9. A femoral prosthesis, according to one or more of the preceding claims, characterized in that said distal body or stem (3) is provided with a distal portion (3a) tapering toward the distal tip portion.

10. A femoral prosthesis, according to one or more of the preceding claims, characterized in that said conic end-piece (6) of said stem is provided with a threaded hole (6a) in which is engaged a screw (16) for connection with said proximal body (2).

## Patentansprüche

1. Eine Femurprothese (1), die speziell zur Wiederherstellung von Prothesenimplantationen entworfen wurde, die zu negativen Ergebnissen geführt haben, wobei diese Femurprothese einen proximalen Körper (2) sowie einen distalen Körper oder Schaft (3) umfaßt, der einen im wesentlichen sternförmigen Querschnitt besitzt, und wobei die Größe des proximalen Bereiches (3b) des Schaftes für sämtliche Längen des Schaftes die gleiche bleibt, wobei dieser proximale Körper (2) an seinem proximalen Endbereich mit einem im wesentlichen elliptischen Querschnitt ausgestattet ist, der sich zu einer im wesentlichen kreisförmigen Gestalt zum distalen Bereich (4) der Spitze hin verändert, dadurch gekennzeichnet, daß in diesem distalen Bereich (4) der Spitze eine konische Aufnahme (5) bereitgestellt ist, um diesen proximalen Körper (2) mit einem konischen Endstück (6) des Schaftes entfernbar zu verbinden, und daß je nach den unterschiedlichen Längen des Schaftes zylindrische, mittlere Bereiche (3c) mit unterschiedlichen Längen bereitgestellt sind.

2. Eine Femurprothese nach Anspruch 1, dadurch gekennzeichnet, daß die Schäfte (3) größerer Länge so gebogen sind, daß sie sich vollkommen an die Krümmung der Femurdiaphyse anpassen.

3. Eine Femurprothese nach den Ansprüchen 1 und 2, dadurch gekennzeichnet, daß entlang der gesamten Längenausdehnung dieser Schäfte (3) größerer Länge diesem im wesentlichen sternförmigen Querschnitt einer oder mehrere Zacken fehlen, die auf den konvexen und konkaven Bereichen dieser Schäfte verteilt sind.

4. Eine Femurprothese nach den Ansprüchen 1 bis 3, dadurch gekennzeichnet, daß dieser proximale Körper in seinem proximalen, seitlichen Bereich einen Ansatz (10) von im wesentlichen dreieckigen Querschnitt besitzt, der sich entlang einer gewissen Länge erstreckt.

5. Eine Femurprothese nach den Ansprüchen 1 bis 4, dadurch gekennzeichnet, daß dieser Ansatz (10) mit einer durchgehenden Öffnung (11) ausgestattet ist.

6. Eine Femurprothese nach einem oder mehreren der vorhergehenden Ansprüche, dadurch gekennzeichnet, daß diese Prothese ferner im proximalen Körper (2) entlang der Ausdehnung dieser konischen Aufnahme (5) einen durchgehenden Kanal (12) umfaßt, um dann eine Schraube (16) zur Verbindung mit dem Endstück (6) des Schaftes (3) aufzunehmen.

7. Eine Femurprothese nach einem oder mehreren der vorhergehenden Ansprüche, dadurch gekennzeichnet, daß in diesem Kanal (12), nämlich in dessen proximalen Endbereich, eine Gewindeaufnahme (14) zur Aufnahme eines Verschlusses (15) darin bereitgestellt ist.

8. Eine Femurprothese nach einem oder mehreren der vorhergehenden Ansprüche, dadurch gekennzeichnet, daß dieser proximale Körper (2), mittig in seinem proximalen Bereich, mit einer ovalen Aufnahme (18) zur Verbindung mit einem Stiel (19) ausgestattet ist, der seinerseits mit einem Kopfteil (20) verbunden wird.

9. Eine Femurprothese nach einem oder mehreren der vorhergehenden Ansprüche, dadurch gekennzeichnet, daß dieser distale Körper oder Schaft (3) mit einem distalen Bereich (3a) ausgestattet ist, der sich zum distalen Bereich der Spitze hin verjüngt.

10. Eine Femurprothese nach einem oder mehreren der vorhergehenden Ansprüche, dadurch gekennzeichnet, daß dieses konische Endstück (6) dieses Schaftes mit einer Gewindebohrung (6a) ausgestattet ist, in die eine Schraube (16) zur Verbindung mit diesem proximalen Körper (2) eingefügt wird.

## Revendications

1. Prothèse fémorale (1), qui a été spécialement conçue pour la restauration d'une implantation prothétique ayant conduit à des résultats négatifs, ladite prothèse fémorale comprenant un corps proximal (2), et un corps distal ou tige (3) ayant sensiblement une section du type en étoile et la dimension de la portion proximale (3b) de la tige étant la même pour toutes les longueurs de la tige, ledit corps proximal (2) ayant, vers sa portion d'extrémité proximale, une section sensiblement elliptique qui se transforme en une configuration sensiblement circulaire en allant vers la portion de pointe ("tip") distale (4), **caractérisée en ce que** dans ladite portion de pointe distale (4) est prévu un logement conique (5) pour accoupler de façon amovible ledit corps proximal (2) avec une pièce d'extrémité conique (6) de la tige, et en ce que, en fonction des différentes longueurs de la tige, sont prévues des portions centrales cylindriques (3c) ayant des longueurs différentes.

2. Prothèse fémorale, selon la revendication 1, **caractérisée en ce que** les tiges (3) de plus grandes longueurs sont courbées de manière à être ajustées précisément à la courbure de la diaphyse fémorale.

3. Prothèse fémorale, selon les revendications 1 et 2, **caractérisée en ce que** tout le long de la longueur desdites tiges (3) de plus grandes longueurs, ladite section sensiblement du type en étoile est dépourvue de un ou plusieurs points qui sont disposés sur les portions convexe et concave desdites tiges.

4. Prothèse fémorale, selon les revendications 1 à 3, **caractérisée en ce que** ledit corps proximal est muni, vers sa région située du côté proximal, d'une aile (10) présentant une section sensiblement triangulaire, et s'étendant le long d'une portion longitudinale.

5. Prothèse fémorale, selon les revendications 1 à 4, **caractérisée en ce que** ladite aile (10) est munie d'un orifice traversant (11).

6. Prothèse fémorale, selon l'une ou plusieurs des revendications précédentes, **caractérisée en ce que** ladite prothèse comprend de plus, à travers le corps proximal (2), sur le prolongement dudit logement conique (5), un canal traversant (12) pour engager en son sein une vis (16) pour la connexion avec la pièce d'extrémité (6) de la tige (3).

7. Prothèse fémorale, selon l'une ou plusieurs des revendications précédentes, **caractérisée en ce que** ledit canal (12), est muni vers sa portion d'extrémité proximale, d'un logement fileté (14) pour engager en son sein un boulon (« plug ») (15).

8. Prothèse fémorale, selon l'une ou plusieurs des revendications précédentes, **caractérisée en ce que** ledit corps proximal (2) est muni, dans sa région médiane proximale, d'un logement oval (18) pour la connexion avec un col (19) qui est à son tour connecté avec une tête (20).

9. Prothèse fémorale, selon l'une ou plusieurs des revendications précédentes, **caractérisée en ce que** ledit corps distal ou tige (3) est muni d'une portion distale (3a) s'effilant vers la portion de pointe ("tip") distale.

10. Prothèse fémorale, selon l'une ou plusieurs des revendications précédentes, **caractérisée en ce que** ladite pièce d'extrémité conique (6) de ladite tige est munie d'un orifice fileté (6a) dans lequel est engagée une vis (16) pour une connexion avec ledit corps proximal (2).
